(19) **Europäisches Patentamt**
**European Patent Office**
**Office européen des brevets**

(11) **EP 2 682 745 B1**

(12) **EUROPEAN PATENT SPECIFICATION**

(45) Date of publication and mention
of the grant of the patent:
**15.05.2019 Bulletin 2019/20**

(51) Int Cl.:
*G01N 33/487* (2006.01)     *A61B 5/145* (2006.01)
*G01N 27/403* (2006.01)

(21) Application number: **12175473.3**

(22) Date of filing: **06.07.2012**

(54) **Monitoring of fluid content**

Überwachung von Flüssigkeitsgehalt

Surveillance du contenu de fluide

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.01.2014 Bulletin 2014/02**

(73) Proprietors:
• **Stichting IMEC Nederland
5656 AE Eindhoven (NL)**
• **Nederlandse Organisatie voor toegepast-
natuurwetenschappelijk onderzoek TNO
2595 DA 's-Gravenhage (NL)**

(72) Inventors:
• **Zevenbergen, Marcel
3086 JE Rotterdam (NL)**

• **Brown, Lindsay
5731 JP Mierlo (NL)**
• **Peter, Maria
5658 GE Eindhoven (NL)**
• **Crego Calama, Mercedes
5663 PK Geldrop-Mierlo (NL)**

(74) Representative: **Haseltine Lake LLP
Redcliff Quay
120 Redcliff Street
Bristol BS1 6HU (GB)**

(56) References cited:
**EP-A1- 2 149 790      EP-A1- 2 237 027
WO-A1-2010/045247      US-A1- 2010 200 400
US-A1- 2012 150 072**

**Description**

This application relates to the monitoring of fluid content, particularly to the content of bodily fluids and especially to methods and apparatus for continuous monitoring of sweat content.

Sweat can contain useful information about the physiological condition of an individual. For instance, it has been shown that both pH and chloride concentration in sweat rise significantly when someone becomes dehydrated. More frequent muscle cramps have been observed in athletes who had a large sodium loss during sweating and lactate levels rose when muscles became exhausted. Sweat monitoring could therefore be used to monitor the condition of an individual, for example to alert to possible dehydration. The content of sweat may also be affected by drug use and thus sweat monitoring could be useful in detecting drug abuse or for monitoring for use of prescribed substances in sport. Analysis of sweat and the content of sweat have also been used in the diagnosis and/or monitoring of a range of medical conditions.

Conventional sweat monitoring operates by collecting a sample of sweat for subsequent analysis. In some applications the subject may be provided with sweat patch which is secured to the skin so as to collect and store sweat. The patch can then be later removed for subsequent analysis. Such methods are useful but only allow periodic analysis of the sweat content and continuous and/or real-time (in-situ) monitoring of sweat contents remains elusive.

Other bodily fluids, such as saliva, may also provide useful information about the condition of an individual.

US 2010/200400 A1 discloses an embedded bodily fluid analysis device comprising a measuring cell containing the bodily fluid to be analyzed, at least one reference cell containing a reference standard and an electronic measuring circuit. US 2012/150072 A1 discloses a device and method for determining an excretion flow rate of a body fluid of a person or an animal.

It is therefore an object of the present invention to provide methods and apparatus for fluid monitoring that at least mitigate some of the above mentioned disadvantages.

Thus according to the present invention there is provided an apparatus for continuous monitoring of sweat content in accordance with claim 1.

Preferred embodiments thereof may comprise the features of dependent claims 2-9.

The invention also relates to a sweat monitoring system comprising said apparatus, as defined in claim 10.

The invention also relates to methods of manufacture of said sweat monitoring apparatus, as defined in claim 11.

The invention will now be described by way of example only, with reference to the accompanying drawings, of which:

Figure 1 illustrates a monitoring apparatus according to an embodiment of the invention suitable for monitoring sweat;

Figure 2 illustrates a method of fabricating a fluid monitoring apparatus according to an embodiment of the invention;

Figure 3 illustrates another method of fabricating a fluid monitoring apparatus according to another embodiment of the invention;

Figure 4 illustrates a fluid monitoring apparatus according to an embodiment of the invention having a plurality of different ion-sensitive electrodes;

Figure 5 illustrates a fluid monitoring apparatus according to an embodiment of the invention including an ion-sensitive electrode layer having an electrode and an ion-selective membrane;

Figure 6 illustrates a fluid monitoring apparatus according to an embodiment of the invention including a reactive layer and sensing electrodes;

Figure 7 illustrates a fluid monitoring apparatus according to an embodiment of the invention with multiple sensing capability;

Figure 8 illustrates a sweat monitoring apparatus having a layer for inducing sweating;

Figure 9 illustrates a fluid monitoring apparatus not in accordance with the present invention, having a sensing layer which reacts with target chemicals to provide a change in physical properties that can be detected; and

Figure 10 illustrates a fluid monitoring apparatus according to a further embodiment of the invention.

In general the sensing apparatus comprises a multilayer structure having at least two electrode layers for detection of fluid content, with the electrode layers being separated by at least one insulating layer. The multilayer structure defines at least one flow channel that provides a flow path for continuous flow of fluid in use. The electrode layers are arranged to form part of the sidewall of the flow channel(s) and thus can analyse the content of the fluid as it flows through the flow channel(s). For monitoring sweat the apparatus may be arranged to be contactable with the body with the flow channel providing a flow path for the fluid from the body.

The multilayer structure can be fabricated as a relatively thin structure that can be applied to a subject as a patch in contact with the subject, for example as a sweat patch for monitoring sweat content. The flow channel(s) may provide a flow path to a surface of the structure that, in use, is exposed to air and/or an absorbent material. The flow channel may therefore provide a flow path for fluid, such as sweat, through the structure to the atmosphere where it can evaporate. The continual evaporation of fluid from the surface of the patch will allow a continuous flow of fluid through the apparatus and past the sensing electrodes. Additionally or alternatively the flow path may be in fluid contact with an absorbent material that absorbs the relevant fluid, e.g. sweat, the action of the absorbent in use resulting in a continual drawing of fluid from the body of the subject through the flow channel(s). In this way the fluid, e.g. sweat, in the vicinity of the sensing electrodes is being constantly replenished without requiring any form of microfluidic system which would significantly add to the cost and complexity of the sensor. This allows the patch to be left in situ to provide continuous monitoring. The analysis of the fluid may thus occur in real time and the sensing electrodes may be connected to suitable readout circuitry. The data acquired could be stored for later analysis which would provide a more complete record of the variation in say sweat content over time than previously possible with conventional sweat monitoring apparatus. However in some embodiments the data may be communicated to a user to provide real-time feedback and/or analysis. The data may be communicated to some data processing circuitry which may be part of the sensing apparatus or part of some other device located on the subject and/or the data may be communicated wirelessly to some remote monitoring device and thus the apparatus may be provided with wireless electronic readout and data transmission circuitry.

The sensor uses the principles of electrochemical detection, for instance for determining the concentration of certain ions in the fluid. Thus the multilayer structure may comprise one or more ion-selective electrodes (which, as will be described, may consist of one or multiple materials or a multilayered structure). These are electrodes generating a voltage scaling with the ion concentration to be determined. The voltage at an ion-selective electrode are compared to a reference electrode which is arranged to be in contact with a constant concentration of the particular ionic species of interest (but otherwise subject to the same operating conditions as the sensing electrode layer). Electrochemical detection for ionic species is known but such detection has not previously been used for continuous/real-time monitoring of sweat content.

For example to detect the concentration of chloride ions in sweat two silver electrodes with a silver chloride surface layer (Ag/AgCl) may be used. One of the electrodes is modified with a coating containing a fixed concentration of chloride ions, for example a polyhydroxyethylmethacrylate (pHEMA) gel, to provide the reference electrode. The voltage of the reference electrode is independent of the ionic content of the sweat. The voltage difference between the two electrodes thus depends on the chloride concentration in the fluid:

$$V = 59mV \log \left[ Cl^- \right] + \text{offset} \qquad \text{Eqn. 1}$$

The sensor can be made sensitive to other ions by adding a semi permeable membrane on top of the other Ag/AgCl electrode, for instance PVC with certain additives tuned to target ionic species.

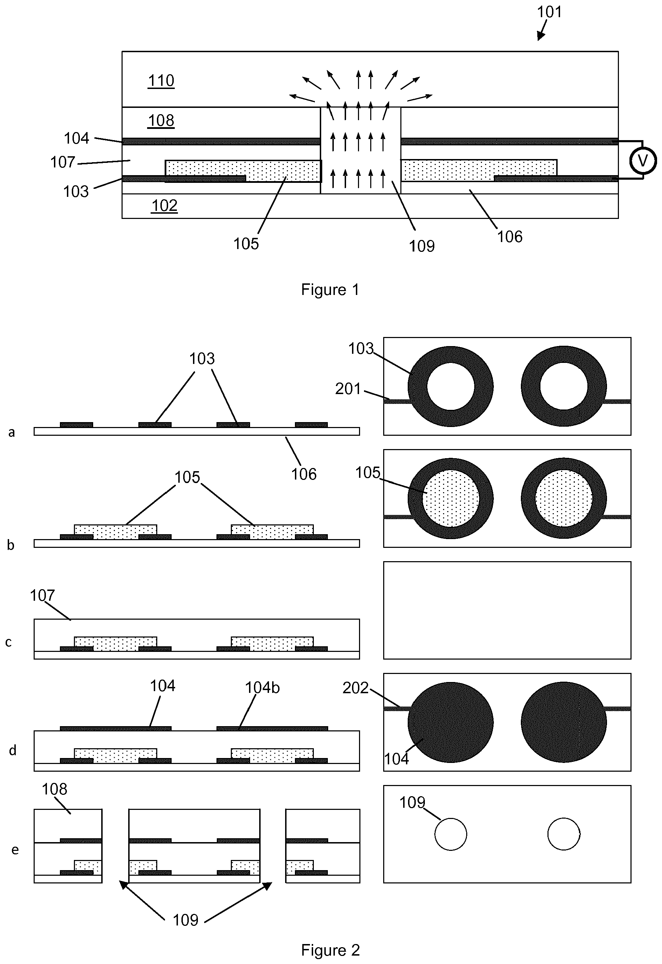

Figure 1 shows a multilayer sensing apparatus 101 according to an embodiment of the invention which is suitable for monitoring of sweat content. The apparatus, in use, is attached to the body of a subject, for instance being attached to the skin 102 of a subject. The multilayer structure comprises a first electrode 103 and a second electrode 104. In this example the first electrode is used as a reference electrode and thus at least part of the first electrode 104 is in contact with a first material 105 which, in use, provides a constant ionic concentration. First electrode 104 and first material 105 together form a first electrode layer which is supported on substrate 106 and separated from the second electrode 104 by at least one insulating layer 107. The second electrode 104 is covered by at least one further insulating layer 108.

A flow channel 109 is provided through the multilayer structure to provide a flow path for the continuous flow of sweat in use. The flow channel is dimensioned so that sweat fills the channel due to capillary action. Optionally an absorbent material layer 110 is positioned on top of the flow channel. The absorbent material, e.g. hygroscopic material, induces a flow in the channel such that the sweat is constantly replenished. In some cases, the absorbent is not required; the channel could be exposed to air and evaporation results in a continuous flow of sweat. The absorbent material could also be embedded in a textile, for instance, were the sweat patch to be included as part of clothing, for instance sports clothing.

The multilayer structure is configured such that the sensing electrode layers form part of the sidewalls of the flow channel. Electrode 103 is deployed as a reference electrode and, as mentioned above is in contact with a first material 105 that provides a constant ionic concentration in use. It is the first material 105 which provides part of the sidewall of the flow channel so that the electrode 103 is not in direct contact with the sweat and thus can act as a reference electrode. Electrode 104 is an ion-sensitive electrode and may form part of the sidewall directly.

In one example the apparatus is used for detecting the concentration of chloride ions in sweat and both electrodes 103 and 104 may be formed from silver coated with silver chloride Ag/AgCl as described above. Material 105 may be any material that provides a fixed concentration of chloride ions in use, such as Poly(2-hydroxyethyl methacrylate) (pHEMA) gel.

In use the voltage between the electrodes 103 and 104 is determined. The voltage difference scales with chloride ion concentration as described above.

As the sensing apparatus comprise a multilayer structure it can be fabricated readily using various printing and/or deposition techniques. The various layers can be relatively thin, for instance an electrode thickness in the side wall of the order of 1 micron or so is sufficient to enable the ionic concentration to be measured. This allows a very thin patch to be produced which can be flexible and can be worn by a subject without discomfort. As mentioned above a multilayer structure of this type could be fabricated as part of an item of clothing.

One method of fabricating a multilayer sensing apparatus will now be described with reference to Figure 2 in which similar components are given the same numerals as figure 1. Figure 2 illustrates formation of a multilayer structure having a plurality of flow channels and shows a series of stages of formation of the structure in section view on the left and plan view on the right.

The method may start by taking a substrate 106 and forming first electrodes 103. The substrate may be any suitable base material such as silicon, glass or foil and may itself comprise a multilayer platform. As the substrate may contact the skin of the subject it may be formed of or coated in some suitable material that is non-irritant. The substrate may also comprise a skin adhesive for adding in attaching to the subject and/or a porous material to provide a route for sweat flow. It will be appreciated however that the substrate may be treated with skin adhesive etc. after device fabrication. Also the substrate does not need to be the layer that contacts the skin in use and the sensor apparatus could be fabricated so that in use the substrate is an outer part of the sensor apparatus.

The electrodes 103 are formed and patterned into a desired shape by for instance, sputtering or evaporating followed by photolithography, or by inkjet printing or screen printing. The electrodes can be formed from any material commonly used in electrochemistry, such as platinum, gold, carbon or diamond although silver is preferred for some embodiments. The electrodes can be of any desired shape. As the electrodes 103 are intended as reference electrodes which will not directly form part of the sidewall of the flow channels the electrodes are patterned so as not to overlap with the area of the eventual flow channels. As an example, electrodes 103 may be patterned to be ring-shaped as illustrated in Figure 2a. The electrodes may also be provided with a conductive track or path 201 to enable electrical connection to the electrode in use.

The electrodes are then, as shown in Figure 2b, at least partly covered by the first material 105 that maintains a constant chloride concentration after exposure to water. As an example, pHEMA gel may be used, which is well known in the state of the art although other materials may also be suitable. The pHEMA gel is patterned, for instance by depositing by known printing techniques, to at least partially cover the electrodes 103 and also to cover part of the substrate 106 in the area which will form the flow channels. In other words the area of the first material 105 does not wholly overlap with the area of the electrode 103.

The multilayer stack is then covered by an insulating layer 107 as shown in figure 2c. The insulating material may be formed from any material or combination of materials that is compatible with the rest of the multilayer apparatus and suitable for the intended application. For instance the insulating layer could comprise silicon dioxide or silicon nitride or a layer of printable material such as polyimide or an insulating epoxy. The layer 107 may itself comprise multiple layers.

Second electrodes 104 and 104b are then formed as shown in figure 2d. The second electrodes may both, for instance, be Ag/AgCl electrodes. The second electrodes are patterned and, in this example, are aligned above the pHEMA layer and the first Ag/AgCl electrode. Again however any shape of second electrode may be used. In this example the second electrodes 104 will form part of the sidewall of the flow channels and thus any pattern in which the second electrodes overlaps the areas of the flow channels would be suitable. Each second electrode 104, 104b may be provided with a conductive path 202 to allow for electrical connections to be made.

Another insulating layer 108 is then deposited and flow channels 109 are formed through the multilayer structure as shown in Figure 2e. The flow channels may be formed by any of a number of techniques such as laser ablation, dry etching techniques, or simple mechanical punching (depending of course on material and channel size). Within the flow channels, part of the second Ag/AgCl electrode 104 is exposed as part of the sidewall, while the first Ag/AgCl electrode 103 is not exposed directly (i.e. the electrode 103 does not itself form part of the sidewall), but indirectly via the pHEMA layer 105, i.e. the first material 105 forms part of the sidewall of the channel. This allows first electrode 103 to form a reference electrode and thus assure correct functioning of the patch.

As mentioned the multilayer structure can be relatively very thin and flexible so as to be easily worn by a subject without discomfort. The exact layer thickness for each layer may depend on the materials used and the desired apparatus characteristics, e.g. strength, rigidity etc. For example to provide a relatively thin structure the various layers may have thicknesses of the order of a few hundred microns or less (although thicker layers may be used in some embodiments if desired). The layers could be much thinner in some embodiments, and may for instance be in the range of about 10nm to several hundred microns.

The width, e.g. diameter, of the flow channels may likewise be of the order of 10nm or so to a few hundred microns. The width of flow channels may depend on the application and the type of fluid being monitored. For instance for a sweat path for use with an athlete where a great deal of sweat is expected in use the flow channels may be relatively wide whereas for monitoring of an elderly patient who would not be expected to sweat as much the flow channels may be relatively less wide. In some embodiments the diameter of the flow channels could be tailored to the sweat rate for the given subject and thus differ for different subjects.

Figure 2 illustrates the basic principles of forming a plurality of flow channels each having (in this example) two electrode layers forming part of the sidewalls of the flow channels. To enable the voltage difference to be determined each of these electrodes would be connected to a readout. Vertical interconnects can be fabricated through the insulating layers to connect conducting paths 201, 202 in the different layers.

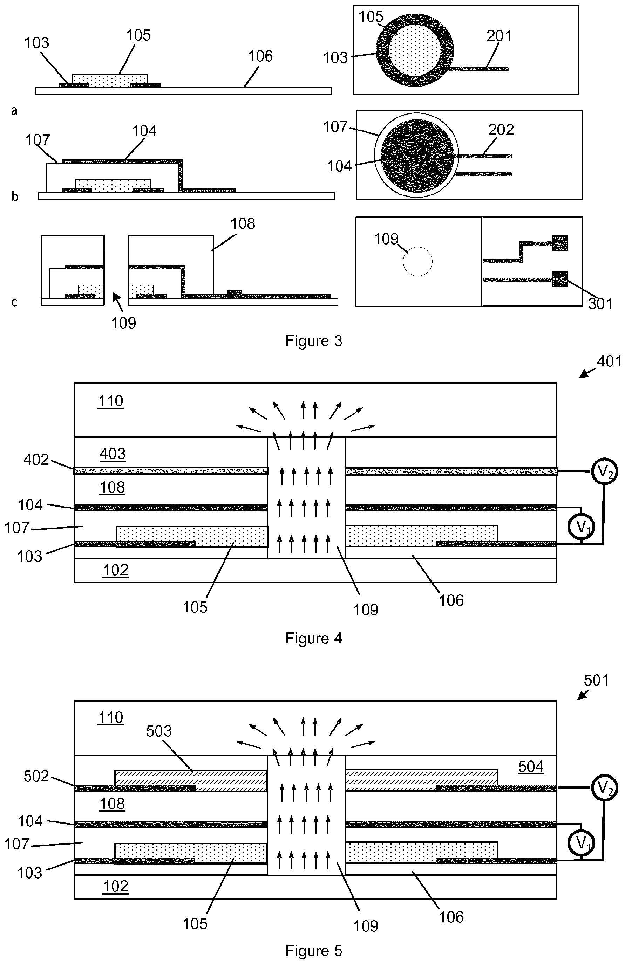

Figure 3 illustrates an alternative way to connect to the electrodes. In this embodiment, as shown in Figure 3a, first electrode 103, and conductive path 201, may be formed on substrate 106 and the electrode partially coated in material 105 that provides a constant ionic concentration as described previously. This electrode layer may then be coated in a layer of insulating material 107 as described previously but in this embodiment, as shown in figure 3b, the insulating layer is patterned to expose the substrate away from the area of the electrode (and hence intended area of the flow channel). For example insulating layer 107 may be formed as a disk shape extending over the first electrode.

The second electrode 104 may then be patterned on top of the insulating disk, for instance as disk, with the conductive trace 202 extending over the edge of insulating layer 107 and connecting to and continuing on the substrate.

The second electrode may then be covered by an additional insulating layer 108 leaving the substrate and ends of conductive connections 201 and 202 exposed as shown in Figure 3c. Flow channel 109 is formed through the multilayer structure as described previously. On the substrate, additional circuitry 301 can be formed. The additional circuitry 301 could simply comprise contacts to allow for connection of other readout circuitry that can measure voltages or currents but in some embodiments the circuitry 301 may comprise readout and/or analysis circuitry, memory and/or wireless communication circuitry. Conveniently, all the steps in the fabrication process are performed by printing processes or standard (photo)lithography methods as known to reduce the manufacturing costs. The substrate may be biodegradable. In this way the fluid monitoring apparatus may be a disposable item.

It will be appreciated that the flow channels 109 described above provide a flow path which is substantially perpendicular to the layers of the multilayer structure. This geometry is particularly advantageous as it allows a very simple and inexpensive fabrication process that can benefit for various multilayer deposition and patterning techniques such as various printing techniques. The multilayer structure can be fabricated by aligning the various electrode layers at a known location and then forming a flow channel through the layers at that location. As only a simple linear channel perpendicular to the layers is needed the channel may, as described above, be formed in a straightforward ablation, etching or punching step.

It will be appreciated however that other geometries would be possible and the flow channel could, if desired, be constructed to run in some other direction and/or to change direction within the multilayer structure, for instance by using sacrificial material to define the flow path during fabrication with subsequent etching or other such techniques. Other geometries are therefore possible albeit involving a more complicated and expensive fabrication process. The use of a multilayer structure with flow channels substantially perpendicular to the layers is a particular advantage of embodiments of the present invention.

The sensing capability of the patch is not limited to sensing chloride only. By using other ion-selective electrodes and measuring the voltages with respect to the reference electrode, other ion-levels can be determined. For instance, referring back to figure 1, by using an iridium oxide electrode as second electrode 104, the sensor could be used to monitor pH level.

In some embodiments however multiple different sensing electrodes may be used to provide an apparatus capable of monitoring for multiple different analytes. The multilayer structure may thus be readily extended by using the same general fabrication techniques to provide a plurality of electrodes for monitoring a plurality of different species.

In one embodiment there are a plurality of ion-selective electrode layers and at least some of the ion-selective electrode layers are sensitive to different target ionic species to one another.

Figure 4 illustrates such an embodiment. Figure 4 shows an apparatus 401 that has all the same layers as the embodiment of figure 1, but in addition to the reference electrode layer (comprising electrode 103 and material 105 that maintains a fixed ionic concentration) and second Ag/AgCl electrode layer 104 there is a third electrode layer 402 and additional insulating layer 403. The third electrode layer may be an iridium oxide electrode which is arranged to directly form part of the sidewall of the flow channel 109. The voltage, V2, between the iridium oxide electrode 402 and reference electrode 103 will give a voltage that directly scales with hydrogen ion concentration, i.e. pH. The multilayer sensor apparatus 401 of figure 4 is thus able to measure both pH and chloride level in a continuous and/or real manner.

Ion-selective electrodes can also be formed by adding a semipermeable membrane in contact with a suitable electrode, for instance a Ag/AgCl electrode. For example, a polyvinylchloride (PVC) membrane with a molecular receptor such as valinomycin or calixarene renders the sweat patch selective to potassium or sodium, respectively. Many ion-selective electrodes for various ions can be formed by changing the composition of the membrane.

Figure 5 illustrates a multilayer sensing apparatus 501 that has all the same layers as the embodiment of figure 1, including the reference electrode layer (comprising electrode 103 and material 105 that maintains a fixed ionic concentration) and second Ag/AgCl electrode layer 104. In this embodiment there is a third electrode layer which comprises a third Ag/AgCl electrode 502 which is in contact with membrane material 503 in such a such as way that the membrane 503 forms part of the sidewall of the flow channel 109. The electrode 502 and membrane 503 are covered by a further insulating layer 504. The third electrode layer may be fabricated in similar way to fabrication of the reference electrode described earlier but using a suitable membrane (such as PVC with suitable molecular receptor) instead of the pHEMA gel. In this example the voltage $V_1$ will scale with chloride concentration and the voltage $V_2$ will scale with a different target ion concentration, such as potassium or sodium.

It will therefore be clear from figures 5 that at least one ion-selective electrode layer may comprise an electrode formed from an electrode material sensitive to a target ionic species where the electrode material itself forms said part of the sidewall of the flow channel, such as the Ag/AgCl electrode 104. Additionally or alternatively at least one ion-selective electrode layer may comprise an electrode in contact with an ion-selective membrane material where it is the ion-selective membrane material (and not the electrode material directly) that forms part of the sidewall of the flow channel, such as Ag/AgCl electrode 502 and membrane 503.

Figures 4 and 5 illustrate providing electrodes with different sensing capability within a single flow channel. Additionally or alternatively the apparatus may be arranged such that there are a plurality of flow channels and at least some flow channels may have different electrode layers to one another to provide different sensing capability. For instance referring back to figure 2 the second electrode 104 formed for one flow channel may be different to the second electrode 104b formed for a different flow channel. For example electrode 104 could be an Ag/AgCl electrode to provide sensing of chloride concentration whereas electrode 104b could be Iridium oxide to provide sensing of pH. In use both flow channels would experience a continual flow of sweat and thus again continuous simultaneous sensing may be provided.

Some compounds of sweat, for instance, lactate, glucose or narcotics or prescribed substances or indicators thereof can be detected by converting the target into an electrochemically active product by an enzyme. As an example, lactate can be converted into pyruvate and hydrogen peroxide by the enzyme lactate oxidase. The hydrogen peroxide can then be detected by a platinum electrode. Similarly, glucose can be detected after conversion by the enzyme glucose oxidase.

The (amperometric) detection mechanism differs compared to operation of the ion-selective electrodes discussed previously. In this instance a voltage is applied between the platinum electrode, referred to as a working electrode, and a reference electrode, while the current flowing between the working electrode and another electrode, the so-called counter electrode is measured.

Suitable enzymes may be immobilized into a porous substrate, for instance a nafion or polycarbonate membrane. In this way reactive layers that act to convert target chemicals into electrochemically active product can be formed and incorporated into a multilayer structure according to an embodiment of the present invention.

In some embodiments of the invention therefore the multilayer structure may comprise at least one reactive layer that reacts with at least one target chemical, if present, to produce an electrochemically active product. The reactive layer may form part of the sidewall of the flow channel downstream of at least one electrode layers which is monitored for the presence of the active product. As mentioned the reactive layer may comprise a porous material having an enzyme immobilized therein that reacts with a target chemical.

The sensing electrodes and reactive layer can be readily incorporated as part of the flow channel in the multilayer structure as described herein. Such a configuration allows for the continuous and real-time determination of a number of additional components of sweat, such as lactate, and thus further extends of the capabilities of the apparatus according to embodiments of the present invention. The reactive layer and corresponding sensing electrodes, i.e. working and counter electrodes, may be used in addition to or instead of the ion-selective electrode layers discussed previously.

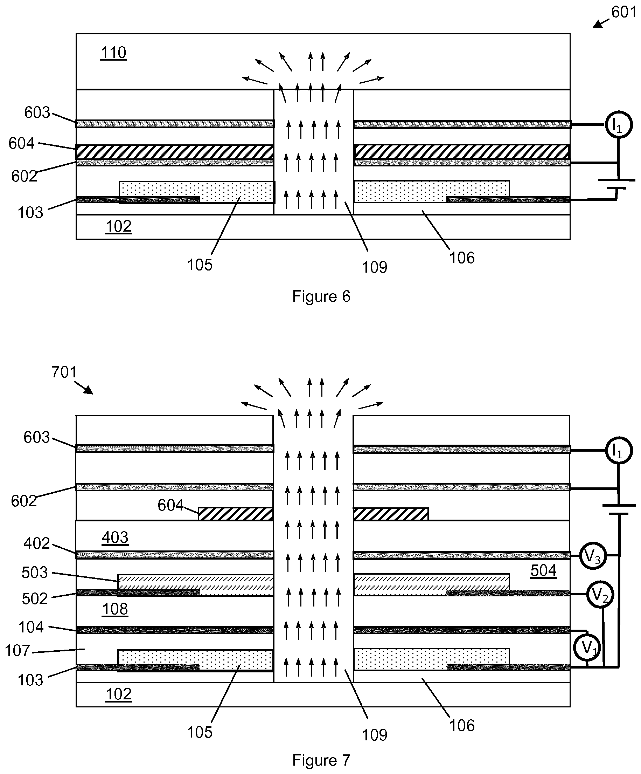

Figure 6 shows a sweat monitoring apparatus 601 according to an embodiment of the invention including a reactive layer that could, for example, be used to determine lactate concentration. The apparatus 601 comprises a reference electrode layer formed from a Ag/AgCl electrode 103 and pHEMA gel as described previously. The embodiment shown in Figure 6 also comprises electrodes 602 and 603 which, in this example, may be platinum electrodes or other commonly used materials in electrochemistry, such as gold, carbon or diamond. Electrodes 602 and 603 are arranged to form part of the sidewall of the flow channel 109.

The multilayer structure 601 also includes a reactive layer 604 which, in this example comprises a porous membrane layer of PVC having enzymes immobilised therein as discussed above. As sweat flow through the flow channel at least some sweat will interact with the reactive layer.

In the embodiment shown in Figure 6 the membrane with the immobilized enzyme is directly fabricated on top of platinum electrode 602. Electrode 603 is formed upstream in the flow path and thus both electrodes 602 and 603 are positioned at suitable parts of the flow path.

In an alternative embodiment however the membrane may be arranged downstream in the flow path of both electrodes 602 and 603 and possibly separated from electrode 602, for example by an insulation layer. In the event of the presence of the target chemical the electrochemically active products will thus be introduced into the rest of the flow path of the flow channel 109 and detected upstream. The configuration used (in terms of location of the reactive layer) may depend on the nature of the enzymes and membranes, for instance whether the enzyme is compatible with a metal electrode.

In use a voltage is applied between the reference electrode 103 and the working electrode, i.e. electrode 602 and the current between the working electrode and the counter electrode, i.e. electrode 603, measured.

The fabrication scheme of the multilayer structure described in effect provides simple building blocks that can be stacked on top of each other to add more parameters to be monitored as desired. The resulting sweat monitoring patch is therefore highly tunable for specific applications.

As an example, an embodiment of a sweat patch which is suitable for the continuous monitoring of pH, lactate, chloride and sodium level is shown in Figure 7. In the embodiment shown in Figure 7 the various sensing layers described previously in other embodiments are identified by the same numerals. Thus Ag/AgCl electrode 103 and pHEMA gel 105 provide a reference electrode. Ag/AgCl electrode 104 is provided to monitor chloride ion concentration. Ag/AgCl electrode 502 is provided with an ion sensitive membrane 503 for detecting sodium ion concentration. Electrode 402 is an iridium oxide electrode for detecting pH level.

Reactive layer 604 comprises a porous membrane having an enzyme for converting lactate into an active product for detection by platinum electrodes 602 and 603. In this embodiment the reactive layer is downstream of the electrode 602 and 603. A potential difference is thus applied between the working electrode 602 and reference electrode 103.

Figure 7 also shows that the flow channel 109 is open to air, although an absorbent could be used as described previously.

The voltages $V_1$, $V_2$, and $V_3$ scale with chloride, sodium and pH, respectively, while the current $I_1$ scales with lactate concentration. Monitoring these parameters is highly desirable for early detection of dehydration or fatigue in sports.

It will of course be understood that the order of electrodes is not fixed and the electrodes could be arranged in a different order if required. The only electrode required for each analyte is the reference electrode, while more functionality can be implemented by adding more electrodes depending on the application. As mentioned previously at least some of the different sensing functions may be implemented by using different electrodes for different flow channels.

It will of course be appreciated that for sweat monitoring apparatus to function correctly the subject should produce enough sweat so as to continually replenish the sweat through the flow channel(s). If the subject is performing reasonable strenuous activity, such as during sports, or is in an environment of elevated temperature the sweat produced by the subject may be sufficient to fill the flow channels in the multilayer patch apparatus by capillary forces. In some applications however it may be wished to monitor the sweat of a subject in situations where the subject is not sweating significantly. For example hospitalized elderly patients do not tend to sweat much but it may be useful to monitor the sweat content of such patients to spot signs of dehydration for example. If the subject does not produce significant sweat the flow channel(s) will not be filled with sweat and/or may dry out. In some embodiments therefore the monitoring apparatus may comprise at least one layer of a material capable of inducing sweating in the subject.

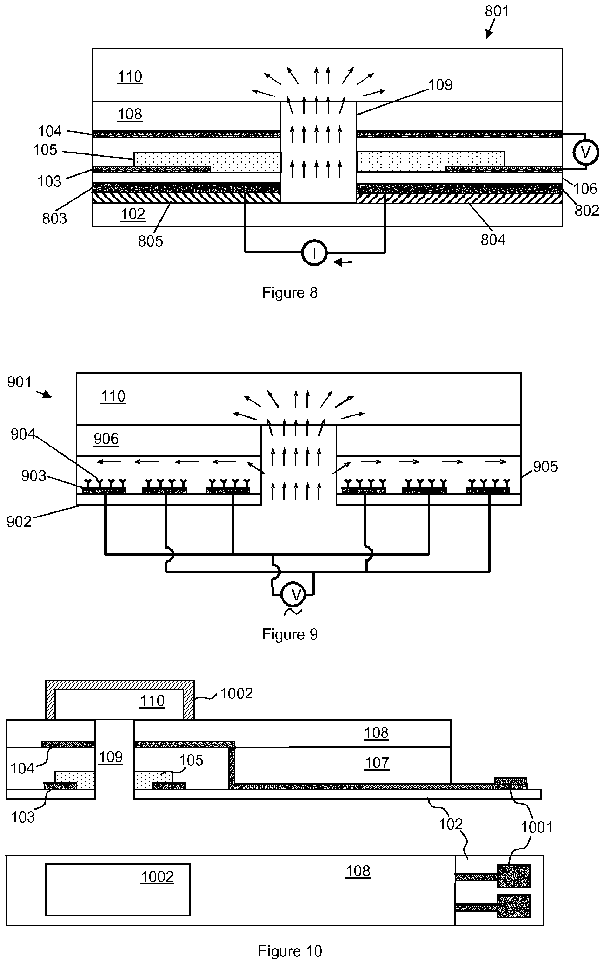

Figure 8 illustrates one embodiment of a multilayer sensing apparatus having means for inducing sweating in a subject. The apparatus has a substrate 106, reference electrode 103 and sensing electrode 104 as described previously. In addition however, on one side of the apparatus (that which contacts the subject in use) there are additional electrodes 802 and 803, at least one of which is modified with a hydrogel 804 containing a chemical such as pilocarpine which can induced sweating. In use the hydrogel 804 is arranged in direct contact with the skin 102 of the subject. Hydrogel 805 containing the inert salt sodium nitrate is also provided adjacent one of the electrodes and also arranged in contact with the skin in use. Pilocarpine is a substance that can induce sweating when it is forced into the skin. This can be achieved by applying a small current of ~1 mA between the electrodes for several minutes. The pilocarpine is forced into the skin by iontophoresis after which the sweating commences. The hydrogel 805 containing sodium nitrate sustains the electric current when sweating is induced.

The sweat monitoring apparatus according to the embodiments of the present invention can be used in a range of applications to provide real time and continuous monitoring. The sweat monitoring patch apparatus may be reusable and, as mentioned, could be integrated into clothing. The apparatus may find application in the field of sports or physical training, for instance providing feedback for personalized uptake of water and salt by athletes and/or for monitoring for use of prescribed substances. Equally the sweat monitoring apparatus could be used in medical fields, for instance for continuous point-of-care dehydration monitoring for elderly and ill people, at home or in hospitals. Whilst the sweat monitoring apparatus is principally useful for monitoring human subjects there may be applications where it is useful to monitor the sweet of an animal that produces sweat, for instance training of race horses or the like.

As mentioned previously the multilayer structure, especially when used with a flow path geometry which is substantially perpendicular to the layers, allows low cost fabrication. All the layers in the fabrication process can be printed to reduce costs, and thus the sweat monitoring patch could be disposable.

In some applications therefore the sweat monitoring apparatus may additionally be used for the single determination of other analytes in sweat that requires antibodies for detection, for instance drugs of abuse, hormones or disease markers. In other words the sweat monitoring apparatus may comprise at least one sensing layer which includes a sensing layer that reacts to the presence of a target chemical to undergo a physical change that can be detected. For instance the physical change may comprise analytes binding to antibodies to change impedance of an electrode. The apparatus may therefore comprise a porous material in contact with at least one electrode treated with a sensing material that reacts with a target chemical, wherein the porous material forms part of the sidewall of the flow channel. In use the sweat will flow through the flow channel and some sweat will be drawn into the porous material. If the target analyte is present the sensing material will react and the presence of the target analyte can be detected.

Figure 9 shows a sweat monitoring apparatus 901 according to this aspect, but not falling within the scope of the present invention. A layer 902 is provided with electrodes 903 modified with sensing material 904. Layer 902 could comprise the top layer (other than the absorbent) of multilayer stack as described in any previous embodiment or an insulating layer within the multilayer stack. The electrodes 903 can be of any shape or material, and may be gold in an interdigitated array design as is shown in Figure 9.

The electrodes 903 are coated or otherwise treated with sensing material 904 which may comprise suitable antibodies for the target chemical. The electrodes 903 and antibodies 904 are covered by an absorbent or other porous layer 905, such as nafion. The porous layer forms part of the sidewall of the flow channel through the multilayer structure and thus, in use, part of the sweat flowing through the channel is absorbed in this layer.

The analyte, if present, reacts with the antibody 904 to form a complex, which is detected for instance by impedance spectroscopy. In this technique an alternating voltage is applied between pairs of electrodes and the impedance is determined.

As the analyte-antibody complex formation is nearly irreversible the sweat monitoring apparatus according to this embodiment of the invention can only be used for a single detection but again provides continuous and real-time monitoring and provides more functionality to the multilayer sweat monitoring capability.

Thus embodiments of the invention relate to fluid monitoring apparatus that may be contactable with a surface at which a fluid of interest may be produced. The apparatus may be attachable to such a surface which may be a surface of the body of a subject. In other embodiments the apparatus may be deployed so as to be immersed in a fluid of interest in use.

Figure 10 shows, in side and plan views, an additional embodiment of an apparatus suitable for being immersed in, or brought into contact with, a fluid of interest by a user. Again components which serve the same purpose as in previous embodiments are reference by the same numerals.

The apparatus is an elongate apparatus with a sensing portion at one end including the flow channel 109 and electrode layers and a handle portion at the opposite end. In the apparatus shown in Figure 10 the substrate 102 and insulating layers 108 and 107 may be elongate to provide the sensing and handle portion but in other embodiments the multilayer apparatus could be mounted to a suitable handle Readout circuitry/contacts 1001 may be located at the handle end of the apparatus which is remote from the flow channel 109.

One end of the flow channel 109 is adjacent an absorbent material 110 as described previously, but in this embodiment the absorbent 110 is located adjacent the substrate 102 The absorbent 110 is contained within a sealing material 1002 which is fluid impermeable such that the only flow path for fluid in use is via the flow channel 109. In use a user may therefore hold the handle end of the apparatus such that the sensing portion is immersed in, or in contact with, the fluid of interest.

Optionally, there may be an additional absorber 1003 in contact with the inlet to the flow channel. This additional absorber may be useful in situations where there is not much fluid in the environment, for example a subject has a relatively dry mouth. The absorber 1003 may draw fluid to the inlet. Once absorber 1003 is relatively saturated capillary action, and the action of the absorber 1003, will draw fluid through the flow channel.

## Claims

1. A sweat monitoring apparatus (101, 401, 501, 601, 701, 801, 901) for continuous monitoring of fluid content comprising:

a multilayer structure comprising at least two electrode layers (103, 104, 402, 502, 602, 603, 802, 803, 902) for detection of fluid content, said electrode layers being separated by at least one insulating layer (107, 108, 403, 504),

wherein the multilayer structure defines at least one flow channel (109), said at least one flow channel (109) providing a flow path for continuous flow of sweat in use, wherein said at least one flow channel (109) runs in a direction substantially perpendicular to said layers, and

wherein said electrode layers form part of the sidewall of said at least one flow channel (109), and one of said electrode layers comprises a reference electrode layer (103, 105) comprising an electrode (103) in contact with a first material (105), wherein the first material (105) does not wholly overlap with the electrode (103) and forms said part of the sidewall of said at least one flow channel (109), the electrode (103) itself does not form part of the sidewall, and the first material provides a substantially constant ionic concentration in use.

2. A sweat monitoring apparatus as claimed in claim 1 wherein at least one electrode layer comprises an ion-selective electrode layer.

3. A sweat monitoring apparatus as claimed in claim 2 wherein at least one ion-selective electrode layer comprises an electrode in contact with an ion-selective membrane material wherein said ion-selective membrane material forms said part of the sidewall of the flow channel.

4. A sweat monitoring apparatus as claimed in any preceding claim wherein said multilayer structure further comprises at least one reactive layer (604) forming part of the sidewall of the flow channel (109) downstream of at least one of said electrode layers, said reactive layer (604) configured to react with at least one target chemical, if present, to produce an electrochemically active product.

5. A sweat monitoring apparatus as claimed in claim 4 wherein at least one reactive layer (604) comprises a porous material having an enzyme immobilized therein that reacts with a target chemical.

6. A sweat monitoring apparatus as claimed in any preceding claim further comprising at least one sensing layer comprising a porous material in contact with at least one electrode treated with a sensing material that reacts with a target chemical, wherein the porous material forms part of the sidewall of the flow channel (109).

7. A sweat monitoring apparatus as claimed in any preceding claim comprising an absorbent material (1003) disposed at one end of the flow channel (109) to draw fluid through the flow channel in use.

8. A sweat monitoring apparatus as claimed in any preceding claim wherein said multilayer structure comprises a plurality of flow channels (109), wherein each flow channel has a plurality of electrode layers forming part of the sidewall of said flow channel (109), wherein the electrode layers vary between at least some flow channels to provide different sensing functionality within said flow channels.

9. A sweat monitoring apparatus as claimed in any preceding claim comprising at least one layer of material capable of inducing sweating.

10. A fluid monitoring system comprising: a sweat monitoring apparatus (101, 401, 501, 601, 701, 801, 901) for continuous monitoring of fluid content as claimed in any preceding claim; and electrical circuitry (1001), wherein said electrical circuitry comprises circuitry for readout of sensing data from said apparatus for continuous monitoring and circuitry for at least one of: storage of said data, transmission of said data and wireless communication.

11. A method of fabricating a sweat monitoring apparatus (101, 401, 501, 601, 701, 801, 901) for continuous monitoring of fluid content comprising:

taking a substrate (106);
forming a plurality of electrode layers (103, 104, 402, 502, 602, 603, 802, 803, 902) over at least part of said substrate, successive electrode layers being separated by at least one insulating layer (107, 108, 403, 504); and

forming at least one channel (109) through said layers and said substrate (106) to define a flow path for continuous flow of sweat in use, such that said electrode layers form part of the sidewall of said at least one channel (109), wherein said at least one channel (109) is formed in a direction substantially perpendicular to the layers;

forming at least one reference electrode layer (103, 105) by forming an electrode (103) in contact with a first material (105), wherein the first material (105) forms said part of the sidewall of said at least one flow channel (109) and provides a substantially constant ionic concentration in use wherein: the first material (105) does not wholly overlap with the electrode (103); and the step of forming the at least one channel (109) comprises forming said channel so that said first material (105) forms part of the sidewall but the electrode (103) itself does not form part of the sidewall.

**Patentansprüche**

1. Schweißüberwachungsvorrichtung (101, 401, 501, 601, 701, 801, 901) zur kontinuierlichen Überwachung des Flüssigkeitsgehalts, umfassend: eine Mehrschichtenstruktur aus mindestens zwei Elektrodenschichten (103, 104, 402, 502, 602, 603, 802, 803, 902) zur Erfassung des Flüssigkeitsgehalts, wobei die Elektrodenschichten durch mindestens eine Isolierschicht (107, 108, 403, 504) getrennt sind, wobei die Mehrschichtenstruktur mindestens einen Strömungskanal (109) definiert, wobei der mindestens eine Strömungskanal (109) im Gebrauch einen Fließweg für den kontinuierlichen Schweißfluss bereitstellt, wobei der mindestens eine Strömungskanal (109) im Wesentlichen senkrecht zu den Schichten verläuft und wobei die Elektrodenschichten einen Teil der Seitenwand von dem mindestens einen Strömungskanal (109) bilden und eine der Elektrodenschichten eine Referenzelektrodenschicht (103, 105) mit einer mit dem ersten Werkstoff (105) in Verbindung stehenden Elektrode (103) umfasst, wobei der erste Werkstoff (105) die Elektrode (103) nicht vollständig überlappt und den Teil der Seitenwand von dem mindestens einen Strömungskanal (109) bildet, wobei die Elektrode (103) selbst keinen Teil der Seitenwand bildet und der erste Werkstoff im Gebrauch eine im Wesentlichen konstante lonenkonzentration liefert.

2. Schweißüberwachungsvorrichtung gemäß Anspruch 1, wobei mindestens eine Elektrodenschicht eine ionensensitive Elektrodenschicht umfasst.

3. Schweißüberwachungsvorrichtung gemäß Anspruch 2, wobei mindestens eine ionensensitive Elektrodenschicht eine mit dem ionensensitiven Membranmaterial in Verbindung stehende Elektrode umfasst, wobei das ionensensitive Membranmaterial einen Teil der Seitenwand des Strömungskanals bildet.

4. Schweißüberwachungsvorrichtung gemäß einem der vorhergehenden Ansprüche, wobei die Mehrschichtenstruktur ferner mindestens eine reaktive Schicht (604) umfasst, die hinter mindestens einer der Elektrodenschichten einen Teil der Seitenwand des Strömungskanals (109) bildet, wobei die reaktive Schicht (604) dafür ausgelegt ist, mit mindestens einer gegebenenfalls vorhandenen Zielchemikalie zu reagieren, um ein elektrochemisch aktives Produkt zu erzeugen.

5. Schweißüberwachungsvorrichtung gemäß Anspruch 4, wobei mindestens eine reaktive Schicht (604) einen mit der Zielchemikalie reagierenden porösen Werkstoff mit einem darin immobilisierten Enzym aufweist.

6. Schweißüberwachungsvorrichtung gemäß einem der vorhergehenden Ansprüche, ferner umfassend mindestens eine einen porösen Werkstoff umfassende Erfassungsschicht, die mit mindestens einer Elektrode in Verbindung steht, die mit einem mit einer Zielchemikalie reagierenden Sensormaterial behandelt wurde, wobei der poröse Werkstoff einen Teil der Seitenwand des Strömungskanals bildet (109).

7. Schweißüberwachungsvorrichtung gemäß einem der vorhergehenden Ansprüche, umfassend einen an einem Ende des Strömungskanals (109) angeordneten absorbierenden Werkstoff (1003), der im Gebrauch eine Flüssigkeit durch den Strömungskanal zieht.

8. Schweißüberwachungsvorrichtung gemäß einem der vorhergehenden Ansprüche, wobei die Mehrschichtenstruktur eine Vielzahl von Strömungskanälen (109) umfasst, wobei jeder Strömungskanal eine Vielzahl von Elektrodenschichten aufweist, die einen Teil der Seitenwand des Strömungskanals (109) bilden, wobei die Elektrodenschichten zwischen mindestens einigen Strömungskanälen unterschiedlich ausgelegt sind, um innerhalb der Strömungskanäle unterschiedliche Sensorfunktionen bereitzustellen.

9. Schweißüberwachungsvorrichtung gemäß einem der vorhergehenden Ansprüche mit mindestens einer Materialschicht, die Schwitzen auslösen kann.

10. Flüssigkeitsüberwachungssystem, umfassend: eine Schweißüberwachungsvorrichtung (101, 401, 501, 601, 701, 801, 901) zur kontinuierlichen Überwachung des Flüssigkeitsgehalts gemäß einem der vorhergehenden Ansprüche;

und eine elektrische Schaltungsanordnung (1001), wobei die elektrische Schaltungsanordnung eine Schaltungsanordnung zum Auslesen von Erfassungsdaten aus der Vorrichtung zur kontinuierlichen Überwachung und eine Schaltungsanordnung für mindestens eine der folgenden Funktionen umfasst: Speicherung der Daten, Übertragung der Daten und drahtlose Kommunikation.

11. Herstellungsverfahren für eine Schweißüberwachungsvorrichtung (101, 401, 501, 601, 701, 801, 901) zur kontinuierlichen Überwachung des Flüssigkeitsgehalts, umfassend: ein Substrat (106) nehmen; mehrere Elektrodenschichten (103, 104, 402, 502, 602, 603, 802, 803, 902) über mindestens einen Teil des Substrats ausbilden, wobei aufeinanderfolgende Elektrodenschichten durch mindestens eine Isolierschicht (107, 108, 403, 504) getrennt sind; und mindestens einen Kanal (109) durch diese Schichten und dieses Substrat (106) bilden, um für den kontinuierlichen Schweißfluss einen Fließweg so zu definieren, dass die Elektrodenschichten einen Teil der Seitenwand von dem mindestens einen Kanal (109) bilden, wobei der mindestens eine Kanal (109) im Wesentlichen senkrecht zu den Schichten ausgebildet ist; mindestens eine Referenzelektrodenschicht (103, 105) durch Bilden einer mit dem ersten Werkstoff (105) in Verbindung stehenden Elektrode (103) bilden, wobei der erste Werkstoff (105) einen Teil der Seitenwand von mindestens einem Strömungskanal (109) bildet und im Gebrauch eine im Wesentlichen konstante Ionenkonzentration bereitstellt, wobei der erste Werkstoff (105) die Elektrode (103) nicht vollständig überlappt; und wobei die Bildung von dem mindestens einen Kanal (109) die so gestaltete Bildung des Kanals umfasst, dass der erste Werkstoff (105) einen Teil der Seitenwand bildet, aber die Elektrode (103) selbst keinen Teil der Seitenwand bildet.

**Revendications**

1. Un appareil de surveillance de la sueur (101, 401, 501, 601, 701, 801, 901) pour la surveillance continue de la teneur en fluide comprenant : une structure multicouche comprenant au moins deux couches d'électrodes (103, 104, 402, 502, 602, 603, 802, 803, 902) pour la détection de la teneur en fluide, lesdites couches d'électrodes étant séparées par au moins une couche isolante (107, 108, 403, 504), dans lequel la structure multicouche définit au moins un canal d'écoulement (109), ledit au moins un canal d'écoulement (109) fournissant un trajet d'écoulement pour l'écoulement continu de la sueur en cours d'utilisation, dans lequel ledit au moins un canal d'écoulement (109) va dans une direction essentiellement perpendiculaire auxdites couches, et dans lequel lesdites couches d'électrodes font partie de la paroi latérale dudit au moins un canal d'écoulement (109), et une desdites couches d'électrodes comprend une couche d'électrodes de référence (103, 105) comprenant une électrode (103) en contact avec un premier matériau (105), dans lequel le premier matériau (105) ne chevauche pas entièrement avec l'électrode (103) et fait partie de ladite paroi latérale dudit au moins un canal d'écoulement (109), l'électrode (103) elle-même ne fait pas partie de la paroi latérale, et le premier matériau fournit une concentration ionique sensiblement constante en cours d'utilisation.

2. Un appareil de surveillance de la sueur selon la revendication 1, dans lequel au moins une couche d'électrode comprend une couche d'électrode sélective d'ions.

3. Un appareil de surveillance de la sueur selon la revendication 2, dans lequel au moins une couche d'électrode sélective d'ions comprend une électrode en contact avec un matériau de membrane sélective d'ions, dans lequel ledit matériau de membrane sélective d'ions fait partie de ladite paroi latérale du canal d'écoulement.

4. Un appareil de surveillance de la sueur selon l'une quelconque des revendications précédentes, dans lequel ladite structure multicouche comprend en outre au moins une couche réactive (604) faisant partie de la paroi latérale du canal d'écoulement (109) en aval d'au moins une desdites couches d'électrodes, ladite couche réactive (604) configurée pour réagir avec au moins un produit chimique cible, si présent, pour produire un produit électrochimiquement actif.

5. Un appareil de surveillance de la sueur selon la revendication 4, dans lequel au moins une couche réactive (604) comprend un matériau poreux ayant une enzyme immobilisée dans celui-ci qui réagit avec un produit chimique cible.

6. Un appareil de surveillance de la sueur selon l'une quelconque des revendications précédentes, comprenant en outre au moins une couche de détection comprenant un matériau poreux en contact avec au moins une électrode traitée avec un matériau de détection qui réagit avec un produit chimique cible, dans lequel le matériau poreux fait partie de la paroi latérale du canal d'écoulement (109).

7. Un appareil de surveillance de la sueur selon l'une quelconque des revendications précédentes, comprenant un matériau absorbant (1003) disposé à une extrémité du canal d'écoulement (109) pour aspirer le fluide à travers le canal d'écoulement en cours d'utilisation.

8. Un appareil de surveillance de la sueur selon l'une quelconque des revendications précédentes, dans lequel ladite structure multicouche comprend une pluralité de canaux d'écoulement (109), dans lequel chaque canal d'écoulement a une pluralité de couches d'électrodes faisant partie de la paroi latérale dudit canal d'écoulement (109), dans lequel les couches d'électrodes varient entre au moins certains canaux d'écoulement pour fournir des fonctionnalités de détection différentes dans lesdits canaux d'écoulement.

9. Un appareil de surveillance de la sueur selon l'une quelconque des revendications précédentes, comprenant au moins une couche de matériau capable de provoquer la sueur.

10. Un système de surveillance de fluide comprenant : un appareil de surveillance de la sueur (101, 401, 501, 601, 701, 801, 901) pour la surveillance continue de la teneur en fluide selon l'une quelconque des revendications précédentes ; et l'ensemble de circuits électriques (1001), dans lequel ledit ensemble de circuits électriques comprend un ensemble de circuits pour l'affichage des données de détection provenant dudit appareil pour une surveillance continue et l'ensemble des circuits pour au moins un de : stockage desdites données, transmission desdites données et communication sans fil.

11. Un procédé de fabrication d'un appareil de surveillance de la sueur (101, 401, 501, 601, 701, 801, 901) pour la surveillance continue de la teneur en fluide comprenant : la prise d'un substrat (106) ; la formation d'une pluralité de couches d'électrodes (103, 104, 402, 502, 602, 603, 802, 803, 902) sur au moins une partie dudit substrat, les couches d'électrodes successives étant séparées par au moins une couche isolante (107, 108, 403, 504) ; et la formation d'au moins un canal (109) à travers lesdites couches et ledit substrat (106) pour définir un trajet d'écoulement pour un écoulement continu de sueur en cours d'utilisation, de sorte que lesdites couches d'électrodes font partie de la paroi latérale dudit au moins un canal (109), dans lequel ledit au moins un canal (109) est formé dans une direction sensiblement perpendiculaire aux couches ; la formation d'au moins une couche d'électrode de référence (103, 105) en formant une électrode (103) en contact avec un premier matériau (105), dans lequel le premier matériau (105) fait partie de ladite paroi latérale dudit au moins un canal d'écoulement (109) et fournit une concentration ionique sensiblement constante en cours d'utilisation, dans lequel : le premier matériau (105) ne chevauche pas entièrement avec l'électrode (103) ; et l'étape de formation de l'au moins un canal (109) comprend la formation dudit canal de sorte que ledit premier matériau (105) fait partie de la paroi latérale mais l'électrode (103) elle-même ne fait pas partie de la paroi latérale.

Figure 1

Figure 2

Figure 3

Figure 4

Figure 5

601

110

603
604
602
103

102

105 109 106

I₁

Figure 6

701

603

602

604

403

402
503
502

108

104
107
103

102

105 109 106

I₁

V₃

504

V₂

V₁

Figure 7

801

110

109

104

105

103

803

802

106

102

805

804

V

I

Figure 8

901

110

906

904

903

902

905

V

~

Figure 9

1002

110

108

104

109

105

107

103

102

1001

1002

108

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010200400 A1
- US 2012150072 A1